# EUROPÄISCHE PATENTSCHRIFT

(11) **EP 4 164 566 B1**
(45) Veröffentlichungstag und Bekanntmachung des Hinweises auf die Patenterteilung: **26.06.2024**
(21) Anmeldenummer: 21733393.9
(22) Anmeldetag: 10.06.2021
(51) Int. Cl.: A61F 13/01, A61F 13/00

(54) **WUNDREINIGUNGSTUCH**
WOUND DEBRIDEMENT CLOTH
LINGETTE DE NETTOYAGE DE PLAIE

(30) Priorität: 10.06.2020 DE 202020103353 U; 03.07.2020 DE 102020117622
(43) Veröffentlichungstag der Anmeldung: 19.04.2023
(73) Patentinhaber: Lohmann & Rauscher GmbH, 2525 Schönau/Triesting (AT)
(72) Erfinder: HÄUSLER, Cornelia, 1230 Wien (AT); TAUSCHER, Carina, 2753 Markt Piesting (AT)
(74) Vertreter: Seranski, Klaus
(86) Internationale Anmeldenummer: PCT/EP2021/065571
(87) Internationale Veröffentlichungsnummer: WO 2021/250148

(56) Entgegenhaltungen:
- WO-A1-2013/113906
- DE-A1-102018 121 501
- DE-U1-202013 104 893
- DE-U1-202019 100 062
- US-A1- 2017 151 314

## Beschreibung

Die Erfindung betrifft ein Wundreinigungstuch nach dem Oberbegriff des Patentanspruchs 1.

Derartige Wundreinigungseinrichtungen sind beispielsweise in der WO 2010/085831 A1 und der PCT/EP/2019/073568 beschrieben. Der Offenbarungsgehalt dieser Schriften hinsichtlich der Eigenschaften der Trägerschicht und der Wundreinigungsschicht sowie der Fäden wird hiermit durch ausdrückliche Bezugnahme in diese Beschreibung aufgenommen.

Wundreinigungstücher der vorstehend beschriebenen Art werden beispielsweise zum sogenannten Debridement eingesetzt. Dabei handelt es sich um den Vorgang der Wundbettpräparation, bei dem vom Körper selbst gebildete Substanzen bzw. Humanmaterial, wie überschießende Flüssigkeit (Wundexsudat), Fibrinbeläge, abgestorbenes Gewebe der Oberhaut, wie zum Beispiel überschießendes Hornmaterial oder tote Hornzellen und/oder Beläge aus abgestorbenem Gewebe (Nekrosen) entfernt werden.

Mit den bekannten Wundreinigungseinrichtungen können die zu entfernenden Substanzen mit Hilfe der Fäden aus der Wunde gelöst und zwischen den Fäden festgehalten werden. Beim Einsatz der bekannten Wundreinigungseinrichtungen hat es sich allerdings gezeigt, dass sich die zu entfernenden Substanzen im Verlauf der Wundreinigung wieder aus der Wundreinigungsschicht lösen und zurück in die Wunde gelangen.

Wundreinigungseinrichtungen nach dem Oberbegriff des Patentanspruchs 1, bei denen ein Speicherstoff in der Trägerschicht der Wundreinigungseinrichtung vorgesehen ist, sind in der WO 2013/113906 A1 angegeben.

In der DE 20 2013 104 893 U1 ist ein superabsorbierende Fasern und superabsorbierende Partikel aufweisender Wundpflegeartikel angegeben.

Die DE 10 2018 121501 A1 offenbart eine Wundreinigungseinrichtung mit strangförmigen Reinigungselementen.

In der DE 20 2019 100 062 U1 sind Wundreinigungseinrichtungen mit einer Wundreinigungsschicht beschrieben, bei der die Wundreinigungsschicht zwei, drei oder mehr Wundreinigungsbereiche mit sich voneinander unterscheidenden Wundreinigungseigenschaften aufweist.

Die US 2017/151314 A1 offenbart ein Debridement-Enzym für nekrotisches Gewebe.

Angesichts dieser Probleme im Stand der Technik liegt der Erfindung die Aufgabe zugrunde, Wundreinigungseinrichtungen mit verbesserter Reinigungswirkung bereitzustellen.

Erfindungsgemäß wird diese Aufgaben durch die im kennzeichnenden Teil des Patentanspruchs 1 angegebene Weiterbildung der bekannten Wundreinigungseinrichtungen gelöst.

Die Erfindung geht auf die Erkenntnis zurück, dass die beim Einsatz der bekannten Wundreinigungseinrichtungen beobachteten Mängel in erster Linie darauf zurückzuführen sind, dass die Aufnahmekapazität der Wundreinigungseinrichtung im Verlauf des Wundreinigungsvorgangs erreicht wird und danach entfernte Substanzen wieder in die Wunde zurückgelangen. Dieser Mangel wird erfindungsgemäß dadurch gelöst, dass geeignete Speicherstoffe eingesetzt werden. Dabei hat sich überraschenderweise gezeigt, dass trotz des von den Speicherstoffen eingenommenen Volumens insgesamt eine erhöhte Speicherkapazität erreicht werden kann, weil einerseits der zur Verfügung stehende Speicherraum der Wundreinigungseinrichtung besser ausgenutzt werden kann und/oder andererseits die Kapazität insgesamt durch geeignete Einbindung des Wundexsudats unter Ausnutzung der Speicherstoffe in die Wundreinigungsschicht und/oder Trägerschicht optimiert wird. Dadurch wird auch verhindert, dass Wundexsudat auf der Umgebungshaut verschmiert und das dadurch verursachte Infektionsrisiko reduziert.

Im Rahmen der Erfindung wird die Aufnahmekapazität der Wundreinigungseinrichtung für im wesentlichen flüssige Stoffe durch die sogenannte "fluid holding capacity" beschrieben. Zur Ermittlung dieser "fluid holding capacity" wird die Wundreinigungseinrichtung zunächst gewogen. Danach wird die Wundreinigungseinrichtung für einen Zeitraum von 60 Sekunden in eine Wundexsudatlösung eingelegt. Anschließend wird das mit der Wundexsudatlösung vollgesogene Produkt mit einer Pinzette ergriffen und für einen Zeitraum von 30 Sekunden in vertikaler Ausrichtung gehalten, so dass Lösung aus der Wundreinigungseinrichtung abtropfen kann. Danach wird das Produkt in horizontaler Ausrichtung in eine Schale eingelegt und erneut gewogen. Anhand der Wägungen wird festgestellt, wie viele Milliliter der Exsudatlösung pro Gramm der Wundreinigungseinrichtung in der Wundreinigungseinrichtung festgehalten werden. Dieser Wert ist die Wundreinigungskapazität im Sinne dieser Erfindung. Falls der Wert durch Zugabe von Speicherstoffen erhöht wird, bedeutet das im Rahmen dieser Erfindung eine Erhöhung der Aufnahmekapazität für Wundexsudate.

Ähnlich wie bei herkömmlichen Wundreinigungseinrichtungen können die Wundreinigungsfäden erfindungsgemäßer Wundreinigungseinrichtungen Kunststofffasern, insbesondere Polyesterfasern und/oder Polypropylenfasern, aufweisen, insbesondere aus Polyesterfasern und/oder Polypropylenfasern bestehen. Dabei können die Reinigungsfäden als Monofilamente, also aus einer Faser pro Faden, und/oder als Multifilamente, also als ggf. verdrillte Faserbündel, ausgeführt sein. Die Reinigungsfäden können im Rahmen der Erfindung Schlaufen bilden, bei denen die Fäden aus der Trägerschicht austreten und nach Schlaufenbildung wieder in die Trägerschicht eintreten. Alternativ oder zusätzlich können die Fäden frei auskragende Enden aufweisen, d. h. dass sie aus der Trägerschicht austreten und frei enden.

Im Rahmen der Erfindung hat es sich als besonders günstig erwiesen, wenn der Anteil der Speicherstoffe, bezogen auf das Trockengewicht von Trägerschicht und Reinigungsschicht (Gesamtgewicht) 0,1 - 25 Gew.-%, insbesondere 0,15 20 Gew.-%, vorzugsweise 0,2 - 17 Gew.-% beträgt. Wenn der Anteil der Speicherstoffe über die genannten Werte erhöht wird, kann dies zu einem Verkleben der Reinigungsfäden führen, was wiederum eine Reduzierung der Speicherkapazität nach sich ziehen kann. Bei einer Verringerung des Anteils der Speicherstoffe unter die genannten Werte ist der Effekt der Speicherstoffe kaum noch bemerkbar.

Es hat sich gezeigt, dass die Speicherstoffe auch innerhalb der Trägerschicht eine die Aufnahmekapazität erhöhende Wirkung entfalten können. Daher hat es sich als zweckmäßig erwiesen, die Menge der Speicherstoffe auf das Gesamtgewicht der Wundreinigungseinrichtung zu beziehen. Allerdings ist auch darauf zu achten, dass die Eigenschaften der Wundreinigungsfäden durch die Speicherstoffe nicht übermäßig beeinträchtigt werden und die verfügbare Aufnahmekapazität zwischen den Reinigungsfäden durch die Speicherstoffe nicht übermäßig reduziert wird. Erfindungsgemäß ist daher vorgesehen, dass der Anteil des Speicherstoffs, bezogen auf das Trockengewicht der Wundreinigungsschicht, 0,2 - 30 Gew.-%, insbesondere 0,25 - 27 Gew.-%, vorzugsweise 0,3 - 25 Gew.-%, beträgt.

Im Rahmen der Erfindung hat es sich als besonders vorteilhaft erwiesen, wenn zumindest die Wundreinigungsschicht mit einer vorzugsweise wässrigen Lösung des Speicherstoffs getränkt ist. So kann ein Verkleben der Reinigungsfäden durch getrocknete Speicherstoffe verhindert werden.

Im Rahmen der beabsichtigten Erhöhung der Speicherkapazität hat es sich als zweckmäßig erwiesen, wenn die Konzentration des Speicherstoffs in der zum Tränken der Reinigungsschicht verwendeten Lösung 0,1 % oder größer, vorzugsweise 0,3 % oder größer und/oder 5 % oder geringer, insbesondere 2,5 % oder geringer, besonders bevorzugt 1,5 % oder geringer ist. Eine Erhöhung der Konzentration bedingt eine erhöhte Viskosität der Lösung. Das kann eine homogene Verteilung der Speicherstoffe in der Reinigungsschicht erschweren. Sofern die Konzentration zu gering ist, wird die verfügbare Kapazität der Reinigungsschicht bereits durch die Tränklösung überwiegend ausgefüllt, so dass dann nur noch eine geringe Restkapazität für das Wundexsudat und die anderen aus der Wunde zu entfernenden Substanzen zur Verfügung steht. Dabei wird die Konzentration angegeben als Gewicht des Speicherstoffs, bezogen auf das Gewicht des Lösungsmittels. Bei Wasser als Lösungsmittel bedeutet eine Konzentration von 0,1 % also 1 g Speicherstoff pro Liter Lösungsmittel.

Im Rahmen der Erfindung hat es sich als besonders zweckmäßig erwiesen, wenn die Viskosität der Lösung auf 2.000 mpas oder weniger, insbesondere 1000 mpas oder weniger, eingestellt wird, gemessen gemäß DIN ISO 2555 mit einem Brookfield-Viskosimeter, Modell RTV.

Der im Rahmen der Erfindung eingesetzte Speicherstoff kann mindestens ein Tensid und/oder mindestens einen bei Kontakt mit Wasser oder einer wässrigen Lösung quellenden Stoff aufweisen. Unter einem Tensid wird im Rahmen der Erfindung eine hydrophilisierende Substanz verstanden, welche hydrophoben Substanzen ermöglicht, in Lösung zu gehen. Tenside bzw. Emulgatoren an sich senken die Energie der Phasengrenze (Oberflächen- oder Grenzflächenspannung). Dies wirkt einer Entmischung entgegen. Die Eigenschaften von Tensiden bzw. Emulgatoren, nicht mischbare Flüssigkeit zu einem feinverteilten Gemisch zu vermengen und zu stabilisieren, fördert im Rahmen der Wundreinigung die gewünschte Erhöhung der Aufnahmekapazität für Wundexsudate. Durch die Reduzierung der Oberflächenspannung mit Hilfe eines Tensids wird auch das Eindringen der Wundexsudate in die Zwischenräume zwischen den Wundreinigungsfäden erleichtert und damit insgesamt die Aufnahmekapazität erhöht.

Bei einem bei Kontakt mit Wasser oder einer wässrigen Lösung quellenden Stoff wird das Wasser in Form eines Gels gebunden. Das führt insgesamt zu einer Erhöhung der Aufnahmekapazität, weil das bereits gebundene Wasser bzw. Wundexsudat nicht wieder aus der Reinigungsschicht heraustropfen kann.

Ein im Rahmen der Erfindung einsetzbarer Speicherstoff kann mindestens einen Celluloseether, insbesondere Hydroxyethylcellulose; Carboxymethylcellulose; Methylcellulose; Ethylcellulose und/oder Hydroxypropylcellulose; weitere Polysaccharide, wie etwa Stärke, Dextrane, Chitosan, Carrageen und Alginate;; Milcheiweiß; Hyaluronsäure; Zuckeralkohole; Glykole; Salze und/oder Mischungen davon aufweisen.

Die Wundheilung kann weiter gefördert werden, wenn die Wundreinigungsschicht und/oder die Trägerschicht mit anti-inflammatorischen und/oder antimikrobiellen Stoffen, wie etwa WO₃ (Wolfram(VI)-oxid) versetzt wird. Dabei wird die antibakterielle Wirkung von WO₃ einer Absorption von Wasser durch das Wolframoxid zugeschrieben, wodurch sich eine dünne, mit H⁺-Ionen angereicherte Schicht bildet, durch die Bakterienmembranen geschädigt werden können. Es ist selbstverständlich möglich, zusätzlich oder alternativ zu WO₃ auch andere antimikrobielle und/oder anti-inflammatorische Stoffe zum Einsatz zu bringen. Dabei kann im Rahmen der Erfindung auch von der antiinflammatorischen/antimikrobiellen Wirkung der Speicherstoffe selbst, wie etwa Hydroxyethylcellulose, Gebrauch gemacht werden.

Im Rahmen der Erfindung ist daran gedacht, Lösungen, insbesondere wässrige Lösungen der Speicherstoffe ggf. mittels eines Sprühflaschenkopfs auf die der Trägerschicht abgewandten Begrenzungsfläche der Wundreinigungsschicht aufzusprühen. Bei alternativen Verfahren wird eine Lösung des Speicherstoffs in gleichmäßigen Abständen in das Pad durch Pipettieren eingebracht. Bei diesem Vorgang erfolgt eine Anfeuchtung der Reinigungsschicht ausgehend von der der Trägerschicht zugewandten Faserbasis. Im Rahmen der Erfindung hat es sich allerdings als besonders zweckmäßig erwiesen, wenn die Speicherstoffe durch Eintauchen der Wundreinigungseinrichtung in eine entsprechende Lösung eingebracht werden. Dabei wird wie folgt vorgegangen:
Bei einer Ausführungsform der Erfindung wird Zellulose als Speicherstoff eingesetzt. Bei Zellulose handelt es sich um das in der Natur am häufigsten verfügbare Polysaccharid mit vielen vorteilhaften Eigenschaften. Dazu gehört die biologische Abbaubarkeit, die Biokompatibilität, fehlende Toxizität, geringe Kosten sowie gute thermische und chemische Stabilität. Die Kombination der Eigenschaften hohe Biokompatibilität mit fehlender Toxizität sowie nicht vorhandener immunogenen Aktivität hat sich im Rahmen der Erfindung als besonders vorteilhaft erwiesen.

Der Speicherstoff, wie etwa Hydroxyethylcellulose (HEC), wird zu destilliertem Wasser zugegeben, bis die gewünschte Konzentration des Speicherstoffs in der Lösung erhalten ist und bei erhöhten Temperaturen von beispielsweise etwa 60° C für eine Stunde kontinuierlich gerührt, um eine homogene Mischung zu erhalten. Bei Ausführungsbeispielen der Erfindung werden Lösungen mit Konzentrationen von 0,3% (3 g/l) und 2,5 % (25 g/l) HEC hergestellt. Bei Einsatz von Hydroxyethylcellulose als Speicherstoff wird 0,5 % Zitronensäure als Vernetzer hinzugefügt. Unter Einsatz von Zitronensäure als Vernetzer kann ein schwammartiges, poröses Material erhalten werden. Diese Eigenschaft wird der starken Wasserstoffbindung zwischen den Polymerketten zugeschrieben. Durch diese starken Wechselwirkungen kann eine sehr große Kapazität zur Absorption großer Mengen von Wasser erhalten werden. Die so erhaltene Struktur kann stark schwellen, ohne in der wässrigen Lösung gelöst zu werden. Die resultierende Lösung wird für 2 Stunden bei erhöhten Temperaturen von beispielsweise etwa 60° C gemischt, um eine Vernetzung zu erreichen und wird ohne vorheriges Abkühlen in eine Glasschale gefüllt. Danach wird die Wundreinigungseinrichtung mit der der Trägerschicht abgewandten Begrenzungsfläche der Wundreinigungsschicht nach unten in die Lösung eingelegt, so dass die Lösung gleichmäßig, ggf. unter Ausnutzung der Kapillarwirkung zwischen den Reinigungsfäden, in der Reinigungsschicht und/oder der Trägerschicht aufgenommen werden kann.

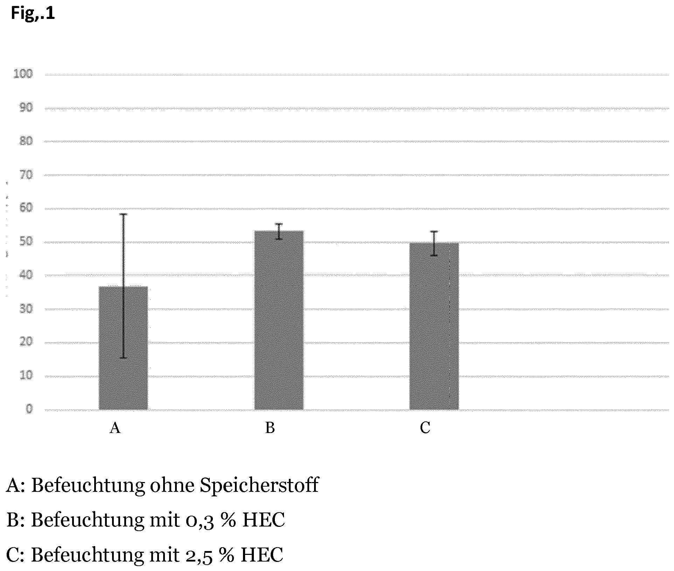

Bei Ausführungsformen der Erfindung können auf der der Trägerschicht abgewandten Begrenzungsfläche der Wundreinigungseinrichtung Monofilamente aus Polyester mit einer Fadenstärke von etwa 1 dtex (1g/10.000 m) und einer Florhöhe (s. u.) von 5 mm vorgesehen sein. Die Masse der Reinigungsfäden (Monofilamente) kann 5 g/100 cm² Fläche betragen. In Figur 1 ist die "fluid holding capacity" der so erhaltenen Wundreinigungseinrichtungen im Vergleich zu ohne Speicherstoff, nur mit destilliertem Wasser vorbefeuchteten Wundreinigungseinrichtungen dargestellt. Angegeben ist die aufgenommene Exsudatmenge bezogen auf das Gewicht der Wundreinigungseinrichtung in Prozent.

Zusätzlich oder alternativ kann die Wundreinigungseinrichtung mit einer ohne besondere Temperaturbehandlung und/oder ohne Einsatz einen Vernetzers erhaltenen Speicherstoff-Lösung versetzt bzw. in eine entsprechende Lösung eingelegt werden.

Im Rahmen der Erfindung ist daran gedacht, die so erhaltene Wundreinigungseinrichtung vor Gebrauch zunächst zu trocknen und erst bei Gebrauch wieder zu befeuchten. Dies kann jedoch dazu führen, dass die Reinigungsfäden miteinander verkleben und die Reinigungswirkung so beeinträchtigt wird. Aus diesem Grund hat es sich im Rahmen der Erfindung als besonders vorteilhaft erwiesen, wenn die Wundreinigungseinrichtung in Form einer Packung mit einer flüssigkeitsundurchlässigen Hülle und einer darin aufgenommenen Wundreinigungseinrichtung, wie etwa einem Wundreinigungstuch, vorliegt. So kann ein Verkleben der Reinigungsfäden während Transport und Lagerung vermieden werden.

Bei einer alternativen Ausführungsform der Erfindung wird ein Kit zum Herstellen einer erfindungsgemäßen Wundreinigungseinrichtung bzw. eines erfindungsgemäßen Wundreinigungstuchs bereitgestellt, mit einer trockenen Wundreinigungseinrichtung bzw. einem trockenen Wundreinigungstuch und einer vorzugsweise wässrigen Lösung mindestens eines Speicherstoffs. Vor Gebrauch der Wundreinigungseinrichtung kann die Wundreinigungsschicht mit der in dem Kit bereitgestellten Lösung getränkt werden, um so eine Ausstattung der Reinigungsschicht und/oder der Trägerschicht mit dem Speicherstoff zu erhalten.

Dabei kann ein erfindungsgemäßes Kit in Form einer sogenannten "Klickpackung" zur Verfügung gestellt werden. In dieser Klickpackung sind Wundreinigungseinrichtung und die Speicherstofflösung in getrennten Kammern aufgenommen. Durch Knicken der Packung an einer vorgegebenen Stelle kann eine Verbindung zwischen den Kammern hergestellt und damit eine Tränkung der Wundreinigungseinrichtung mit der Speicherstofflösung herbeigeführt werden.

Im Rahmen der Erfindung hat sich der Einsatz von Hydroxyethylcellulose als Speicherstoff als besonders zweckmäßig erwiesen. Dieser Stoff ist hitzebeständig und eignet sich auch für Sterilisationsverfahren unter Einsatz hoher Temperaturen. Bei Einsatz dieses Speicherstoffs hat es sich als besonders bevorzugt erwiesen, wenn die Wundreinigungseinrichtung mit einer wässrigen Lösung des Speicherstoffs befeuchtet und danach in einer flüssigkeitsundurchlässigen Hülle gelagert wird. Hydroxyethylcellulose kann als wässrige Lösung in einem Konzentrationsbereich von üblicherweise zwischen 0,1 und 1,5 % verwendet werden. Im Rahmen der Erfindung ist es besonders zweckmäßig, wenn Hydroxyethylcelluloselösungen mit Konzentrationen zwischen 0,1 und 5 % zum Tränken der Wundreinigungseinrichtung verwendet werden. Bei höheren Konzentrationen wird die Lösung sehr viskos und kann kaum noch aufgetragen werden.

Bei Einsatz von Hydroxyethylcellulose als Speicherstoff kann von der Eigenschaft der Hydroxyethylcellulose als muco-adhesives Polymer Gebrauch gemacht werden. Dies bedeutet, dass es sehr gut an Schleimhäute bindet. Da die Oberflächenbeschaffenheit von Wunden und Schleimhäuten als ähnlich angesehen werden kann, haftet Hydroxyethylcellulose ebenso gut an Wundoberflächen. Dadurch wird eine entsprechende Filmbildung gefördert. Der Hydroxyethylcellulosefilm verhindert die Austrocknung der Wunde bzw. führt dazu, dass Feuchtigkeit in der Wunde gehalten werden kann, und fördert so die Wundheilung.

Bei einer erfindungsgemäßen Wundreinigungseinrichtung können die Reinigungsfäden als Flor vorliegen. Dabei kann die Flormasse, das heißt die Masse der Reinigungsschicht, 1 - 10 g/100 cm² Fläche betragen. Die Reinigungsschicht kann in diesem Fall Monofilamente aus Polyester aufweisen, welche zwischen 0,5 und 20 dtex aufweisen können. Die Fäden können von der Trägerschicht abstehende Fäden mit frei auskragenden Enden aufweisen, wobei die Fäden auch einen gekrümmten Verlauf besitzen können. Die Länge der außerhalb der Trägerschicht für die Wundreinigung verfügbaren Fadenabschnitte (Florhöhe) kann 0,1 - 50 mm, insbesondere 1-40 mm, vorzugsweise 3-30 mm, besonders bevorzugt 4-12 mm, wie etwa 5 mm oder 10 mm, betragen. Sie müssen auch nicht senkrecht von der Trägerschicht abstehen. Die Trägerschicht kann ein Vlies, Gewebe oder eine Maschenware aufweisen. Alternativ oder zusätzlich kann die Trägerschicht auch als Membran oder Film ausgeführt sein, in dem die Reinigungsfäden teilweise aufgenommen sind. Hinsichtlich der Herstellung von im Rahmen der Erfindung einsetzbaren Wundreinigungseinrichtungen bzw. -tüchern wird Bezug genommen auf die WO 2010/085831 A1, welche auch in dieser Hinsicht durch ausdrückliche Inbezugnahme in diese Beschreibung aufgenommen wird.

Zusätzlich oder alternativ zu Hydroxyethylcellulose können erfindungsgemäße Wundreinigungseinrichtungen unter anderem mit den folgenden Speicherstoffen versehen sein, wobei Speicherstofflösungen zum Einsatz kommen können, die ähnlich hergestellt werden wie für Hydroxyethylcellulose beschrieben und die Wundreinigungseinrichtung durch Einlegen in entsprechende Speicherstofflösungen, Aufsprühen und/oder Pipetieren mit dem Speicherstoff ausgestattet werden kann:

### Weitere Tenside

Poloxamere sind Blockcopolymere aus Ethylenoxid und Propylenoxid. Es ist ein reinigendes und antimikrobielles Tensid. Poloxamer 188 erlangt durch Propoxlierung eine erhöhte Hitzestabilität (bis 260° C), welche die Substanz kompatibel mit üblichen Sterilisationsverfahren unter Hitze macht. Neben der erhöhten Hitzestabilität können durch die Propoxylierung Konservierung, Antioxidation, Antischaumbildung und ein Viren abtötender Effekt erreicht werden. Optional kann Butylhydroxytoluol zugesetzt werden. Als weitere Tenside können im Rahmen der Erfindung eingesetzt werden Tween-20/Tween-180, Esterquats, Natriumdodecylsulfat, Betain, EHG (Ethylhexylglycerin), Carboxymethylcellulose, Methylcellulose, Ethylcellulose, Hydroxypropylcellulose.

### Milcheiweiß

Zur Herstellung erfindungsgemäßer Wundreinigungseinrichtungen kann beispielsweise Milchpulver (Fett/Eiweiß/Milchzucker) eingesetzt werden. Dabei wirkt Milcheiweiß ebenfalls als Emulgator.

### Säuren

Säuren weisen antimikrobielle Eigenschaften auf. Der natürliche pH-Wert der Haut liegt mit einem Wert von 4,5 bis 5,5 im leicht sauren Bereich. Grund dafür ist, dass ein leicht saures Milieu die Haut dabei unterstützt, Krankheitserreger von außen abzutöten. Im Rahmen der Erfindung können zur Herstellung erfindungsgemäßer Wundreinigungseinrichtungen Essigsäure, Milchsäure, Zitronensäure; Ameisensäure und dergleichen eingesetzt werden.

Hyaluronsäure ist feuchtigkeitsspendend; es bilden sich Wasserbrückenbildung aus (wasserbindend) und die O-Spannung wird herabgesetzt. Da die Hyaluronsäure bedeutend für den Austausch von Informationen zwischen den Zellen ist, hat sie ebenso im Prozess der Wundheilung einen positiven Effekt. Sie sorgt dafür, dass Wunden heilen, ohne sichtbare Narben zu hinterlassen. Das kann im Rahmen dieser Erfindung ausgenutzt werden.

### Zuckeralkohole

Zuckeralkohole, wie etwa Glycerol, weisen wasserbindende Eigenschaften auf. Die wasserbindende Wirkung kann für die gesteigerte Aufnahme von Wundexsudat genutzt werden. Es wurde unter Einsatz von Zuckeralkoholen, insbesondere von Glycerol, auch eine antimikrobielle Wirkung beobachtet.

### Glycole

Im Rahmen der Erfindung können für die Herstellung erfindungsgemäßer Wundreinigungseinrichtungen auch Glycole, wie PEG 500/3000 (Polyethylenglycol) eingesetzt werden. PEG ist wasserlöslich und stark hygroskopisch. Die Fähigkeit, Wasser aufzunehmen, kann im Rahmen der Erfindung zur Kapazitätserhöhung von Wundreinigungseinrichtungen genutzt werden. Zusätzlich zeigt PEG auch einen antimikrobiellen Effekt.

### Salze

Im Rahmen der Erfindung kann von der geruchsbindenden, desinfizierenden und wasserbindenden Wirkung von Salzen Gebrauch gemacht werden. Salze dienen im Rahmen der Erfindung als Speicherstoff. Sie können zusätzlich antimikrobielle Eigenschaften aufweisen und den Geruch von Wunden neutralisieren. Im Rahmen der Erfindung können als Speicherstoff eine Ringerlösung, bestehend aus Natriumchlorid, Kaliumchlorid und Calciumchlorid, Laktate (Salze und Ester der Milchsäure), Natriumacetat (Natriumsalz der Essigsäure), Kochsalz und Salze von Säuren eingesetzt werden. Es können auch Gemische der vorstehend erläuterten Speicherstoffe eingesetzt werden.

### Palmitoyl Oligopeptid

Dieser im Rahmen der Erfindung einsetzbare Speicherstoff, ein natürliches Tripeptid, weist eine biologische Wirkung im Zusammenhang mit der Neubildung von Glycosaminoglykanen und Kollagenen auf und führt zu einer erhöhten Hydrierung.

## Patentansprüche

1. Wundreinigungseinrichtung mit einer Trägerschicht und einer an dieser angeordneten, zum Aufnehmen von Wundexsudat und/oder festen Wundbestandteilen ausgelegten sowie Reinigungsfäden aufweisenden Wundreinigungsschicht, wobei die Wundreinigungseinrichtung mit mindestens einem die Aufnahmekapazität für Wundexsudat erhöhenden Speicherstoff versehen ist, **dadurch gekennzeichnet, dass** die Wundreinigungsschicht mit dem Speicherstoff versehen ist und der Anteil des mindestens einen Speicherstoffs bezogen auf das Trockengewicht der Reinigungsschicht 0,2 - 30 Gew.-%, insbesondere 0,25 - 27 Gew.-%, vorzugsweise 0,3 - 25 Gew.-% beträgt.

2. Wundreinigungseinrichtung nach Anspruch 1, **dadurch gekennzeichnet, dass** die Reinigungsfäden Kunststofffasern, insbesondere Polyesterfasern aufweisen, insbesondere daraus bestehen.

3. Wundreinigungseinrichtung nach Anspruch 1 oder 2, **dadurch gekennzeichnet, dass** die Reinigungsfäden als Monofilamente und/oder Multifilamente ausgeführt sind.

4. Wundreinigungseinrichtung nach einem der vorhergehenden Ansprüche, **dadurch gekennzeichnet, dass** die Reinigungsfäden Schlaufen bilden und/oder frei auskragende Enden aufweisen.

5. Wundreinigungseinrichtung nach einem der vorhergehenden Ansprüche, **dadurch gekennzeichnet, dass** der Anteil des mindestens einen Speicherstoffs bezogen auf das Trockengewicht von Trägerschicht und Reinigungsschicht 0,1 - 25 Gew.-%, insbesondere 0,15 - 20 Gew.-%, vorzugsweise 0,2 - 17 Gew.-% beträgt.

6. Wundreinigungseinrichtung nach einem der vorhergehenden Ansprüche, **dadurch gekennzeichnet, dass** zumindest die Reinigungsschicht mit einer vorzugsweise wässrigen Lösung des Speicherstoffs getränkt ist.

7. Wundreinigungseinrichtung nach Anspruch 6, **dadurch gekennzeichnet, dass** die Konzentration des Speicherstoffs in der Lösung 0,1 % oder mehr, vorzugsweise 0,3 % oder mehr und/oder 5 % oder weniger, insbesondere 2,5 % oder weniger, besonders bevorzugt 1 % oder weniger beträgt.

8. Wundreinigungseinrichtung nach einem der vorhergehenden Ansprüche, **dadurch gekennzeichnet, dass** der Speicherstoff mindestens ein Tensid und/oder mindestens einen bei Kontakt mit Wasser oder einer wässrigen Lösung quellenden Stoff aufweist.

9. Wundreinigungseinrichtung nach einem der vorhergehenden Ansprüche, **dadurch gekennzeichnet, dass** der Speicherstoff mindestens einen Celluloseether, insbesondere Hydroxyethylcellulose, Carboxymethylcellulose, Methylcellulose, Ethylcellulose und/oder Hydroxypropylcellulose, weitere Polysaccharide, wie etwa Dextrane, Stärke, Chitosan, Carrageen und Alginate, Milcheiweiß, Hyaluronsäure, Zuckeralkohole, Glykole, Salze und/oder Mischungen davon aufweist.

10. Verfahren zum Herstellen einer Wundreinigungseinrichtung nach einem der vorhergehenden Ansprüche, bei dem zumindest die Wundreinigungsschicht der Wundreinigungseinrichtung mit einer vorzugsweise wässrigen Lösung des Speicherstoffs getränkt wird.

11. Packung mit einer flüssigkeitsundurchlässigen Hülle und einer darin aufgenommenen Wundreinigungseinrichtung nach einem der Ansprüche 1 bis 9.

12. Kit zum Herstellen einer Wundreinigungseinrichtung nach einem der Ansprüche 1 bis 9 mit einem trockenen Wundreinigungstuch und einer vorzugsweise wässrigen Lösung mindestens eines Speicherstoffs.

## Claims

1. A wound debridement device comprising a carrier layer and, arranged thereon, a wound debridement layer configured to hold wound exudate and/or solid wound components and having debridement threads, wherein the wound debridement device is provided with at least one storage material that increases the holding capacity for wound exudate, **characterised in that** the wound debridement layer is provided with the storage material and the proportion of the at least one storage material, based on the dry weight of the debridement layer, is 0.2-30% by weight, in particular 0.25-27% by weight, preferably 0.3-25% by weight.

2. The wound debridement device according to claim 1, **characterised in that** the debridement threads contain plastic fibres, in particular polyester fibres, and in particular consist thereof.

3. The wound debridement device according to claim 1 or 2, **characterised in that** the debridement threads are configured as monofilaments and/or multifilaments.

4. The wound debridement device according to one of the preceding claims, **characterised in that** the debridement threads form loops and/or have freely projecting ends.

5. The wound debridement device according to one of the preceding claims, **characterised in that** the proportion of the at least one storage material, based on the dry weight of the carrier layer and debridement layer, is 0.1-25% by weight, in particular 0.15-20% by weight, preferably 0.2-17% by weight.

6. The wound debridement device according to one of the preceding claims, **characterised in that** at least the debridement layer is impregnated with a preferably aqueous solution of the storage material.

7. The wound debridement device according to claim 6, **characterised in that** the concentration of the storage material in the solution is 0.1% or more, preferably 0,3% or more and/or 5% or less, in particular 2.5% or less, particularly preferably 1% or less.

8. The wound debridement device according to one of the preceding claims, **characterised in that** the storage material contains at least one surfactant and/or at least one substance that swells on contact with water or with an aqueous solution.

9. The wound debridement device according to one of the preceding claims, **characterised in that** the storage material contains at least one cellulose ether, in particular hydroxyethyl cellulose, carboxymethyl cellulose, methyl cellulose, ethyl cellulose and/or hydroxypropyl cellulose, additional polysaccharides, such as dextrans, starch, chitosan, carrageenan and alginates, milk protein, hyaluronic acid, sugar alcohols, glycols, salts and/or combinations thereof.

10. A method for producing a wound debridement device according to one of the preceding claims, in which at least the wound debridement layer of the wound debridement device is impregnated with a preferably aqueous solution of the storage material.

11. A pack comprising a fluid-impermeable wrapper and, accommodated therein, a wound debridement device according to one of claims 1 to 9.

12. A kit for producing a wound debridement device according to one of claims 1 to 9, comprising a dry wound debridement cloth and a preferably aqueous solution of at least one storage material.

## Revendications

1. Dispositif de débridement de plaie comportant une couche de support et, sur celle-ci, une couche de débridement conçue pour absorber l'exsudat de la plaie et/ou des constituants solides de la plaie, et comportant également des fils de débridement, le dispositif de débridement étant pourvu d'au moins un matériau d'accumulation qui accroit la capacité d'absorption d'exsudat; **caractérisé en ce que** la couche de débridement est dotée dudit matériau d'accumulation et la proportion de l'au moins un matériau d'accumulation rapportée au poids sec de la couche de débridement est de 0,2 à 30 % en poids, notamment de 0,25 à 27 % en poids et de préférence de 0,3 à 25 % en poids.

2. Dispositif de débridement selon la revendication 1, **caractérisé en ce que** les fils de débridement comprennent des fibres plastiques, plus particulièrement des fibres de polyester, et se composent notamment de celles-ci.

3. Dispositif de débridement selon la revendication 1 ou 2, **caractérisé en ce que** les fils de débridement consistent en des monofilaments et/ou multifilaments.

4. Dispositif de débridement selon l'une des revendications précédentes, **caractérisé en ce que** les fils de débridement forment des boucles et/ou présentent des extrémités librement saillantes.

5. Dispositif de débridement selon l'une des revendications précédentes, **caractérisé en ce que** la proportion de l'au moins un matériau d'accumulation rapportée au poids sec de la couche de support et de la couche de débridement est de 0,1 à 25 % en poids, notamment de 0,15 à 20 % en poids, de préférence de 0,2 à 17 % en poids.

6. Dispositif de débridement selon l'une des revendications précédentes, **caractérisé en ce qu'**au moins la couche de débridement est imbibée d'une solution, de préférence aqueuse, dudit matériau d'accumulation.

7. Dispositif de débridement selon la revendication 6, **caractérisé en ce que** la concentration en matériau d'accumulation dans la solution est de 0,1 % ou plus, de préférence de 0,3 % ou plus et/ou de 5 % ou moins, notamment de 2,5 % ou moins, de préférence de 1 % ou moins.

8. Dispositif de débridement selon l'une des revendications précédentes, **caractérisé en ce que** le matériau d'accumulation présente au moins un tensioactif et/ou au moins une substance susceptible de gonfler au contact de l'eau ou d'une solution aqueuse.

9. Dispositif de débridement selon l'une des revendications précédentes, **caractérisé en ce que** le matériau d'accumulation comprend au moins un éther de cellulose, notamment de l'hydroxyéthylcellulose, de la carboxyméthylcellulose, de la méthylcellulose, de l'éthylcellulose et/ou de l'hydroxypropylcellulose, d'autres polysaccharides, tels que dextranes, amidon, chitosane, carraghénane et alginates, des protéines de lait, de l'acide hyaluronique, des alcools de sucre, des glycols, des sels et/ou des mélanges de ceux-ci.

10. Procédé de réalisation d'un dispositif de débridement selon l'une des revendications précédentes, dans lequel au moins la couche de débridement du dispositif de débridement est imbibée d'une solution, de préférence aqueuse, dudit matériau d'accumulation.

11. Ensemble comportant une enveloppe imperméable aux liquides renfermant un dispositif de débridement selon l'une des revendications 1 à 9.

12. Kit de réalisation d'un dispositif de débridement selon l'une des revendications 1 à 9, comportant une lingette sèche de débridement et une solution, de préférence aqueuse, d'au moins un matériau d'accumulation.
